# EUROPEAN PATENT APPLICATION

(11) **EP 1 607 116 A2**
(43) Date of publication of application: **21.12.2005**
(21) Application number: 05011447.9
(22) Date of filing: 27.05.2005
(51) Int. Cl.: A61M 5/50, A61M 5/32

(54) **Safe syringe with automatic retractable needle**

(30) Priority: 16.06.2004 CN 200420066060
(71) Applicant: Tung, Chung-Hao, Taipai (TW)
(72) Inventor: Tung, Chung-Hao, Taipai (TW)
(74) Representative: Meyer, Ludgerus

(57) **Abstract**

The safe syringe comprises a cylinder (1), a needle seat (2) and a pusher plunger (3). The needle seat (2) is firmly connected to the front inner side of the cylinder (1), the needle seat (2) has on its bottom (21) a first protruding piercing portion (22), a chamber (24) with a positive pressure is formed between the bottom (21) of the needle seat (2) and the front end of the cylinder (1); the pusher plunger (3) is a hollow rod having a chamber (31) therein with a negative pressure, the rod has a second piercing portion (33) on the outer wall (32) on its front end, and a piston (4) envelops the front surface (34) of the rod and the outer wall (32). Thereby, when the plunger (3) is pushed to the front end of the cylinder (1), the first piercing portion (22) pierces and breaks the piston (4) and the front surface (34) of the rod, the second piercing portion (33) pierces forwards through and cut out the bottom (21) of the needle seat (2) to make communication of the two chambers (24,31), thus the bottom (21) of the needle seat (2) is drawn into the plunger (3) together with the needle (23) by changing of pressure.

## Description

### BACKGROUND OF THE INVENTION

### 1. Field of the invention

The present invention is related to a safe syringe with an automatic retractable needle, and especially to a safe syringe on which a chamber with a positive pressure is formed between the bottom of a needle seat and the front end of a cylinder, and another chamber with a negative pressure is formed in a pusher plunger, so that the two chambers can be communicated with each other when the pusher plunger is pushed to the front end of the cylinder, and in turn the needle is drawn into the pusher plunger by changing of pressure. The syringe is structurally simple, convenient for operation; and when the needle is retracted into the pusher rod, it has no worry of safety concerning dropping out of the cylinder. The safe syringe suits medical therapies.

### 2. Description of the Prior Art

In processes of medical treatment, to inject medicines into the bodies of patients for controlling the situations of diseases is a therapy often seen; and to inject medicines with syringes has been the most effective mode of therapy and has been adopted by the medical field. Used needles have contacted patients, if the diseases of the patients are infective, the needles becomes infective too, it must be very cautious and careful in dealing with such needles to prevent medical staffs or personnel discarding wastes from being hurt by the infective needles.

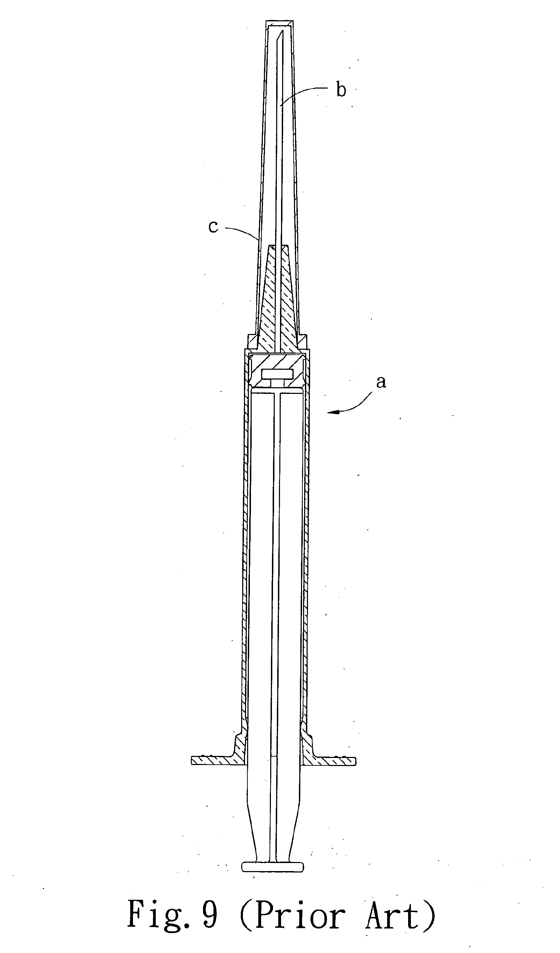

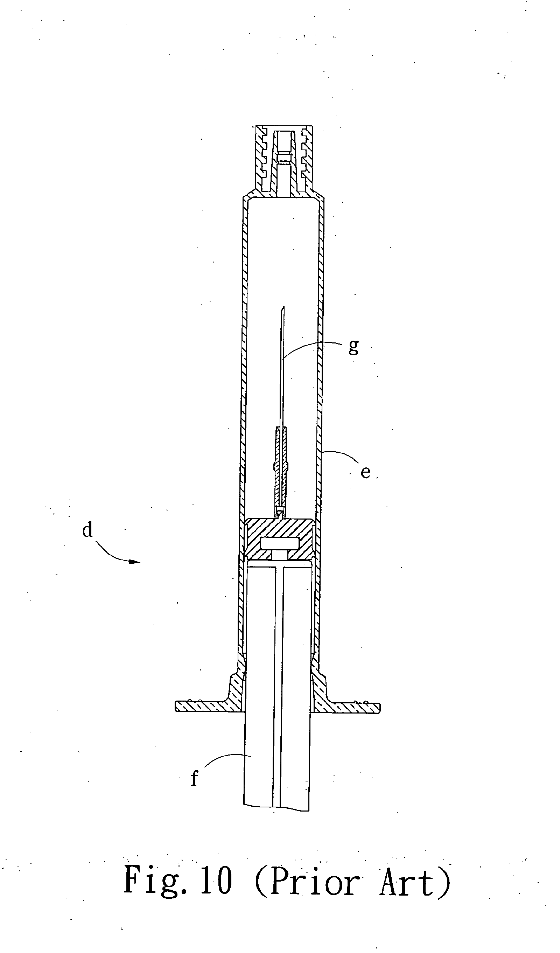

As shown in Fig. 9, a needle "b" of a syringe "a" of an earlier stage is only covered by a cover "c" to prevent one from being hurt by it; however, the cover "c" is designed in pursuance of the needle "b", its opening for slipping over the needle "b" is quite small, and thereby it shall be accurately aligned with the needle "b" for slipping over the latter. In view of this, for the safety of medical staffs, designs for syringes with various retractable type needles and protecting means for injection needles have been developed. Such as is shown in Fig. 10, a so-called retractable type syringe "d" allows a medical staff to push a plunger "f" of its cylinder "e" to an uppermost position to draw a needle "g" back into the cylinder "e", in order that the syringe "d" being discarded after use can be dealt with safety.

However, safe syringes with retractable type needles in the markets presently still have the following defects although they can achieve the object of drawing back the needles:
1. Designing of a safe syringe with a retractable type needle mainly provides a means for mutual engaging (such as of an engaging hook with an engaging detent) between a needle seat and a plunger; when a piston of the plunger is pushed to an end, the engaging hook is very easy to engage with the engaging detent, when the two are engaged with each other, the needle seat and the needle can be together drawn back into a cylinder of the syringe. This structural design and the production for it are very complicated, and the costs of them are relatively raised.
2. When in use, every medical staff must use two hands to draw back the needle seat and the needle into the cylinder, and the plunger can be pulled out together with the needle directly by an unduly exerted force, such syringes are inconvenient and dangerous in use; moreover, when the syringe are discarded randomly or a staff deals with the discarded syringe in a neglectful attitude to render the plunger to be pushed out of the cylinder after the needle has been drawn back into the cylinder, the retractable type will lose its meaning of designing.
3. After using of the safe syringe with the retractable type needle, the volume of the plunger will mostly be exposed to the outside by pulling out of the cylinder, it is increased abruptly in length, thus the volume for treatment during discarding is also increased.

In view of these, the inventor develops a safe syringe with an automatic retractable needle to overcome the defects resided now popularly in the above stated safe syringes with retractable type needles.

### SUMMARY OF THE INVENTION

The primary object of the present invention is to provide a safe syringe with an automatic retractable needle that takes advantage of a vacuum negative pressure and a positive pressure to generate a suction force and a pushing force to render a needle to automatically retract into a pusher plunger.

Another object of the present invention is to provide a safe syringe with an automatic retractable needle that is structurally simple, convenient for operation, and the needle has no worry of safety concerning dropping out of the cylinder after drawing back.

To achieve the above objects, the safe syringe with an automatic retractable needle of the present invention is comprised of a cylinder, a needle seat and a pusher plunger. Wherein the cylinder is a hollow barrel having a needle extending-through cone at the center on the front end thereof; the needle seat is firmly connected to the inner side of the front end of the cylinder, the needle seat has on the bottom thereof a first protruding piercing portion and has a needle, the needle is partially extended out of the needle extending-through cone, a chamber with a positive pressure is formed between the bottom of the needle seat and the front end of the cylinder; the pusher plunger is a hollow rod, able to move forwards and rearwards in the cylinder to draw in medicine for injection, the interior of the rod is a chamber with a negative pressure, the rod has a second piercing portion on the outer wall on the front end thereof, and a piston envelops the front surface of the rod and the outer wall.

Thereby, when the plunger is pushed to the front end of the cylinder, the first protruding piercing portion provided on the bottom of the needle seat pierces and breaks the piston at the front end of the pusher plunger and the front surface of the rod, the second piercing portion on the outer wall on the front end of the rod pierces forwards through and cut out the bottom of the needle seat to make mutual communication of the two chambers, the bottom of the needle seat is drawn into the pusher plunger together with the needle by changing of pressure.

The present invention will be apparent after reading the detailed description of the preferred embodiments thereof in reference to the accompanying drawings.

### BRIEF DESCRIPTION OF THE DRAWINGS

Fig. 1 is an analytic perspective view showing the appearance of an embodiment of the present invention;
Fig. 2 is a schematic sectional view of the embodiment of the present invention after assembling;
Fig. 3 is a schematic sectional view of the embodiment of the present invention showing a cylinder contains medicine before injection;
Fig. 4 is a schematic sectional view of the embodiment of the present invention showing the state that a first piercing portion pierces a piston;
Fig. 5 is a schematic sectional view of the embodiment of the present invention showing the state that a second piercing portion pierces and cut out the bottom of a needle seat;
Fig. 6 is a schematic sectional view of the embodiment of the present invention showing the bottom of the needle seat is drawn into a pusher plunger together with a needle;
Fig. 7 is a schematic sectional view of the embodiment of the present invention showing the pusher plunger has been completely pushed to the front end of the cylinder;

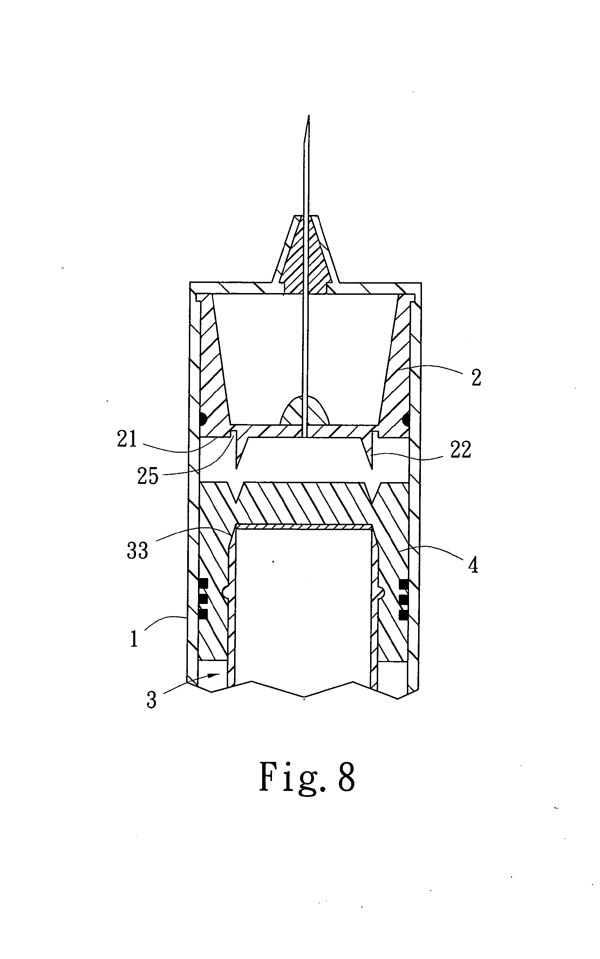

Fig. 8 is a partial schematic sectional view of another embodiment of the present invention showing a needle seat is provided around a first piercing portion with a recess;
Fig. 9 is a sectional view showing a conventional needle is slipped thereover with a cover;
Fig. 10 is a schematic sectional view of a conventional retractable type safe syringe showing the needle is drawn back into a cylinder in use.

### DETAILED DESCRIPTION OF THE PREFERRED EMBODIMENT

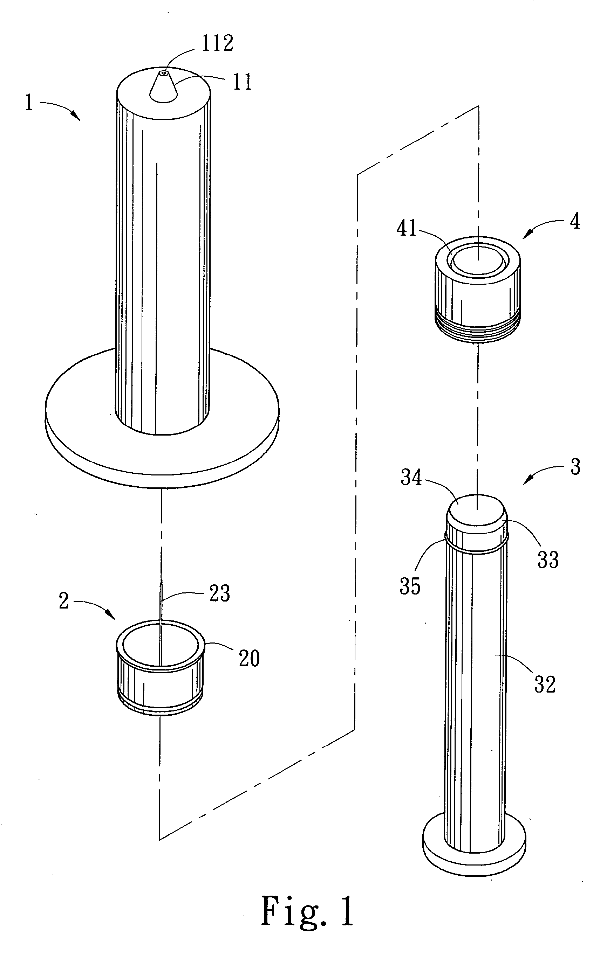

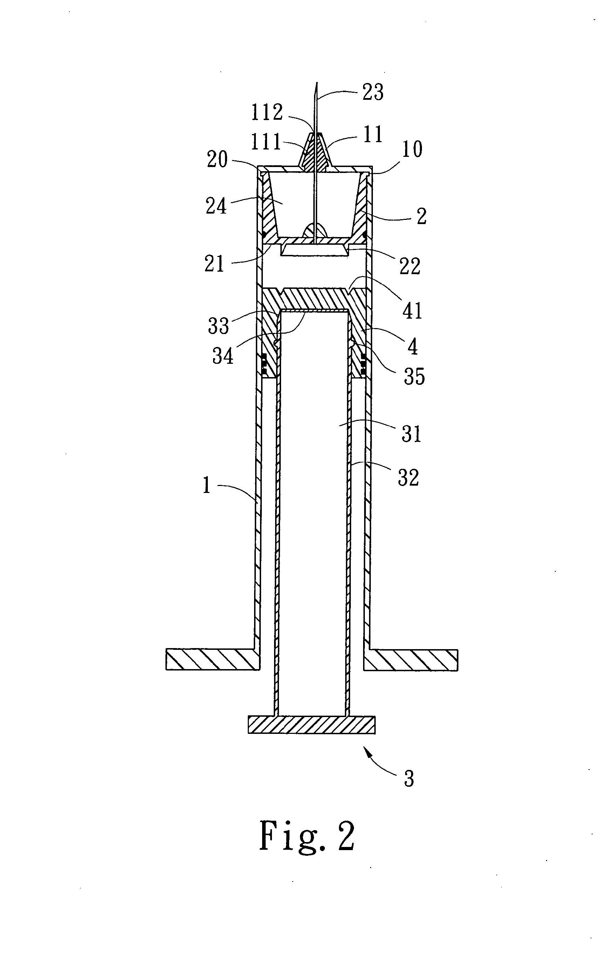

Referring to Figs. 1, 2 depicting an embodiment of a safe syringe with an automatic retractable needle of the present invention, the syringe comprises: a cylinder 1, a needle seat 2 and a pusher plunger 3.

The cylinder 1 is a hollow barrel having in the front end thereof a connecting portion 10, and having a needle extending-through cone 11 at the center on the front end; the needle extending-through cone 11 protrudes out of the front end of the cylinder 1, and is filled therein with a sealing rubber layer 111 having therein a passageway 112.

The needle seat 2 has an outer connecting member 20 to connect with the connecting portion 10, after then, the needle seat 2 is firmly connected to the inner side of the front end of the cylinder 1, the needle seat 2 has on the bottom 21 thereof a first piercing portion 22; the first piercing portion 22 is annular and is raised from the bottom 21 of the needle seat 2, its outer diameter is smaller than the outer diameter of the pusher plunger 3; the needle seat 2 has a needle 23, the needle 23 is partially extended out of the needle extending-through cone 11 through the passageway 112, a chamber 24 with a positive pressure is formed between the bottom 21 of the needle seat 2 and the front end of the cylinder 1.

The pusher plunger 3 is a hollow rod for slipping in the cylinder 1, and can move forwards and rearwards in the cylinder 1 to draw in medicine for injection, the interior of the rod 3 is a chamber 31 with a negative pressure, the rod 3 has a second piercing portion 33 on the outer wall 32 on the front end thereof, and a piston 4 envelops the front surface 34 of the rod 3 and the outer wall 32; the second piercing portion 33 has an annular sharp edge formed by inwardly tapering of the outer wall 32 on the front end of the pusher plunger 3, the outer wall 32 of the pusher plunger 3 has a protruding ring 35 at the upper section of the second piercing portion 33 to make firm of the connection of it with the piston 4, the piston 4 is provided on its bottom with another recess 41 for piercing of the first piercing portion 22.

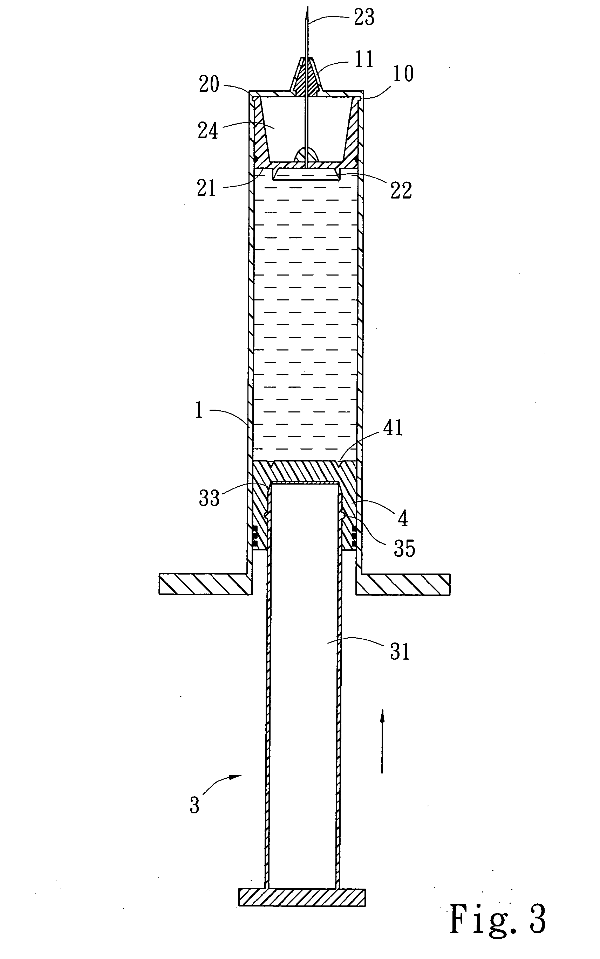

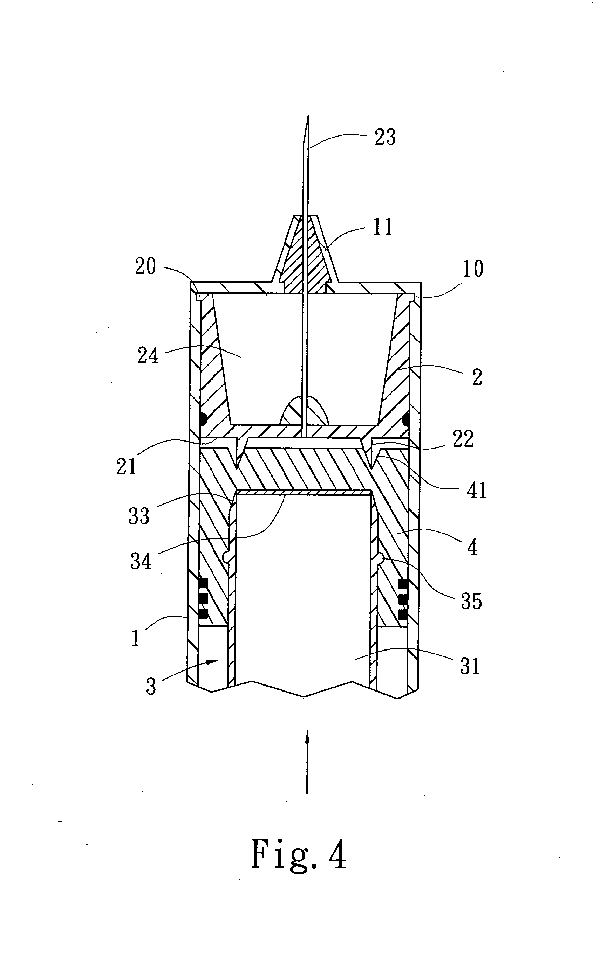

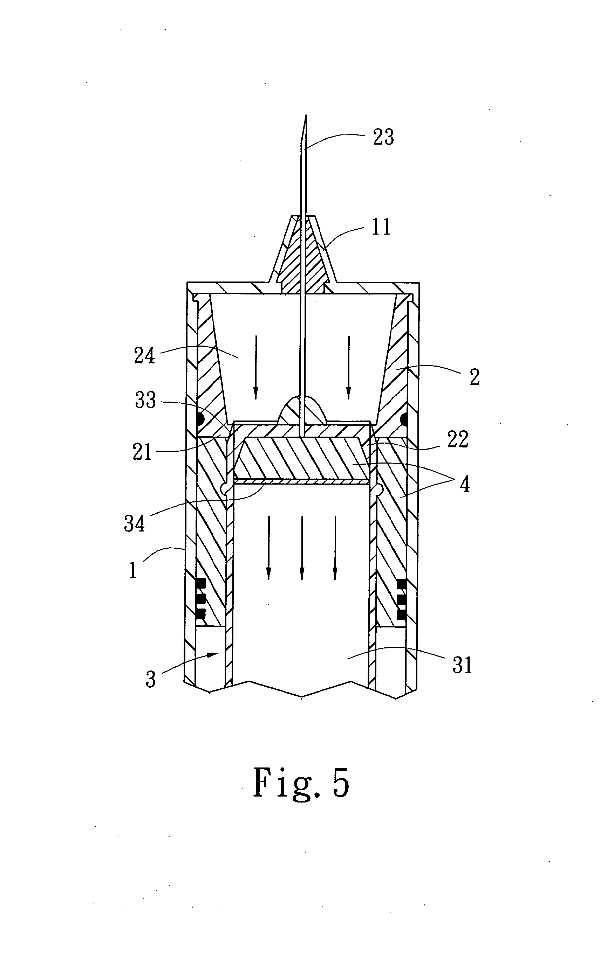

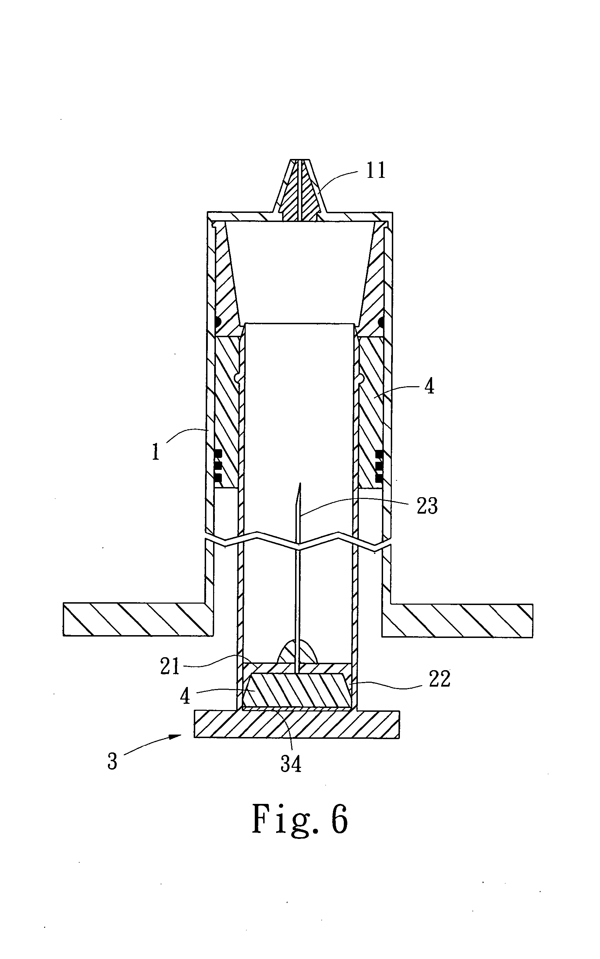

Referring to Figs. 2-6, in manufacturing, the interior of the front end of the cylinder 1 is provided with an engaging groove forming the connecting portion 10, and the needle seat 2 has on the outer side thereof an engaging flange forming the outer connecting member 20; when the bottom 21 of the needle seat 2 and the front end of the cylinder 1 have therebetween the chamber 24, the needle seat 2 is firmly connected to the inner side of the front end of the cylinder 1, therefore, after completion of injection of medicine and drawing of a syringe out of the body of a patient, the pusher plunger 3 can be further pushed to the front end of the cylinder 1 (as shown in Figs. 3 and 4), the piston 4 enveloping the front surface 34 of the pusher plunger 3 and the outer wall 32 exactly contacts the first piercing portion 22 extending upwards from the bottom 21 of the needle seat 2, so that the first piercing portion 22 exactly can extend in from the recess 41 provided on the bottom of the piston 4 to break the piston 4 and the front surface 34 of the pusher plunger 3, the second piercing portion 33 on the outer wall 32 on the front end of the rod 3 pierces forwards and cut out the bottom 21 of the needle seat 2 (as shown in Fig. 5), now the piston 4 is received respectively within the first piercing portion 22 and also surrounds the outside of the latter, while the chamber 31 with a negative pressure is communicated with the chamber 24 with a positive pressure by virtue that the front surface 34 of the pusher plunger 3 and the bottom 21 of the needle seat 2 are respectively subjected to breaking by the first piercing portion 22 and the second piercing portion 33, then the bottom 21 of the needle seat 2 is drawn into the pusher plunger 3 together with the needle 23 by changing of pressure (as shown in Fig. 6).

Thereby, the needle 23 can be received in the pusher plunger 3, when the pusher plunger 3 is pushed completely to the front end of the cylinder 1, the protruding ring 35 provided on the pusher plunger 3 is exactly engaged in the chamber 24 of the needle seat 2 to form interference, thus the pusher plunger 3 is no more able to be drawn away the cylinder 1, and the needle 23 can be sealed in the pusher plunger 3 without a problem of dropping.

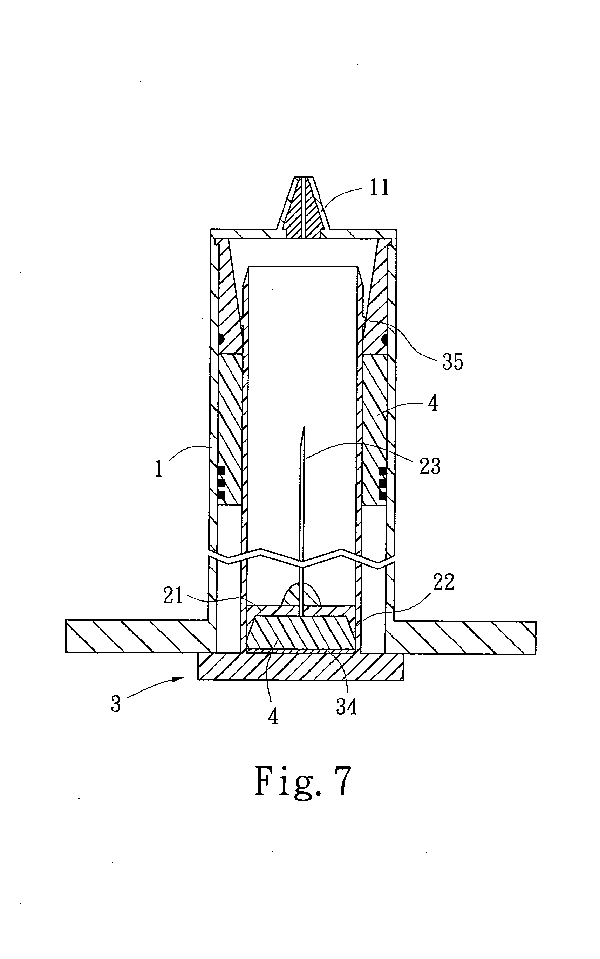

Further, as shown in Fig. 7, in order that the second piercing portion 33 can be easier to break the bottom 21 of the needle seat 2, the needle seat 2 can be provided around the first protruding piercing portion 22 with a recess 25 to be pierced by the second piercing portion 33.

The present invention thereby has the following advantages:
1. In the present invention, a chamber with a positive pressure is formed between the bottom of a needle seat and the front end of a cylinder, and another chamber with a negative pressure is formed in a pusher plunger, so that two chambers can be communicated with each other when the pusher plunger is pushed to the front end of the cylinder to create a drawing force and a pushing force, and in turn the needle is drawn into the pusher plunger. The syringe is structurally simple; it can effectively reduce its cost of production.
2. The present invention renders the needle to automatically retract into the pusher plunger by pushing the pusher plunger to the front end of the cylinder, an operator no more needs a pulling force to draw back the needle; such operation is convenient and energy saving, and can avoid the problem that the plunger is pulled out together with the needle directly by an unduly exerted force; and the plunger is still left in the cylinder, this can reduce the volume for dealing with during discarding.
3. The present invention has a protruding ring on the pusher plunger convenient for moving of the pusher plunger, the protruding ring can be engaged in the chamber of the needle seat to form interference when the pusher plunger is completely pushed to the front end of the cylinder after injection of the syringe, thereby the pusher plunger is no more able to be drawn away the cylinder, and the needle can be sealed in the pusher plunger without a problem of dropping.

As stated in the above disclosed, the present invention can surely attain its expected objects to provide a safe syringe with an automatic retractable needle; it is extremely industrial valuable.

## Claims

1. A safe syringe with an automatic retractable needle, said syringe comprises:
a cylinder, being a hollow barrel having a needle extending-through cone at the center on a front end thereof;
a needle seat firmly connected to an inner side of said front end of said cylinder, said needle seat has on the bottom thereof a first protruding piercing portion and has a needle, said needle is partially extended out of said needle extending-through cone, a chamber with a positive pressure is formed between said bottom of said needle seat and said front end of said cylinder; and
a pusher plunger being a hollow rod adapted for slipping in said cylinder, able to move forwards and rearwards in said cylinder to draw in medicine for injection, the interior of said pusher plunger is a chamber with a negative pressure, said pusher plunger has a second piercing portion on an outer wall on a front end thereof, and a piston envelops a front surface of said pusher plunger and said outer wall; thereby, when said pusher plunger is pushed to said front end of said cylinder, said first protruding piercing portion provided on said bottom of said needle seat pierces and breaks said piston at said front end of said pusher plunger and said front surface of said pusher plunger, said second piercing portion on said outer wall on said front end of said pusher plunger pierces forwards through and cut out said bottom of said needle seat to make mutual communication of said two chambers, said bottom of said needle seat is drawn into said pusher plunger together with said needle by changing of pressure.

2. The safe syringe with an automatic retractable needle as claimed in claim 1, wherein said needle extending-through cone protrudes out of said front end of said cylinder, and is filled therein with a sealing rubber layer having therein a passageway.

3. The safe syringe with an automatic retractable needle as claimed in claim 1, wherein said cylinder has in said front end thereof a connecting portion, said needle seat has an outer connecting member to connect with said connecting portion, after then, said needle seat is firmly connected to the inner side of said front end of said cylinder.

4. The safe syringe with an automatic retractable needle as claimed in claim 3, wherein the interior of said front end of said cylinder is provided with an engaging groove forming said connecting portion, and said needle seat has on the outer side thereof an engaging flange forming said outer connecting member.

5. The safe syringe with an automatic retractable needle as claimed in claim 1, wherein said first piercing portion is annular and is raised from said bottom of said needle seat, its outer diameter is smaller than an outer diameter of said pusher plunger; spaces are formed within said first piercing portion and surrounds the outside of said first piercing portion to receive said piston.

6. The safe syringe with an automatic retractable needle as claimed in claim 1, wherein said needle seat is provided around said first protruding piercing portion with a recess to be pierced by said second piercing portion.

7. The safe syringe with an automatic retractable needle as claimed in claim 1, wherein said piston is provided on its bottom with a recess for piercing of said first piercing portion.

8. The safe syringe with an automatic retractable needle as claimed in claim 1, wherein said outer wall of said pusher plunger has a protruding ring at an upper section of said second piercing portion to make firm of connection of it with said piston; when said pusher plunger is pushed to said front end of said cylinder, said protruding ring is engaged in said needle seat, thus said pusher plunger is restrained in said cylinder.

9. The safe syringe with an automatic retractable needle as claimed in claim 1, wherein said second piercing portion has an annular sharp edge formed by inwardly tapering of said outer wall on said front end of said pusher plunger.
